# EUROPEAN PATENT APPLICATION

(11) **EP 1 589 115 A2**
(43) Date of publication of application: **26.10.2005**
(21) Application number: 05011183.0
(22) Date of filing: 17.08.2001
(51) Int. Cl.: C12Q 1/68, C07K 14/47, C07K 16/18

(54) **Polymorphisms of the human organic anion transporting polypeptide-C (OATP-C)**

(30) Priority: 23.08.2000 US 226909 P
(62) Divisional of application: 01306983.6
(71) Applicant: AstraZeneca AB, 151 85 Södertälje (SE)
(72) Inventor: Adeokun, Monisola, Macclesfield Cheshire, SK10 4TG (GB); Ambrose, Helen Jean, Macclesfield Cheshire, SK10 4TG (GB); Cresswell, Carl John, Macclesfield Cheshire, SK10 4TG (GB); Dudley, Adam Jeston, Wilmington DE 19850-5437 (US)
(74) Representative: Giles, Allen Frank

(57) **Abstract**

This invention relates to polymorphisms in the human OATPC gene and corresponding novel allelic polypeptides encoded thereby. The invention also relates to methods and materials for analysing allelic variation in the OATPC gene, and to the use of OATPC polymorphism in treatment of diseases with OATPC transportable drugs such as statins.

## Description

This invention relates to polymorphisms in the human OATPC gene and corresponding novel allelic polypeptides encoded thereby. The invention also relates to methods and materials for analysing allelic variation in the OATPC gene, and to the use of OATPC polymorphism in treatment of diseases with OATPC transportable drugs.

Na+-independent organic anion transporting polypeptide (OATP) C gene is a member of the OATP supergene family involved in multifunctional transport of organic anion. OATPC tranports the organic anion taurocholate, conjugated steroids: DHEAS, estradiol 17p-D-glucoronide and estrone-3-sulfate, eicosanoids: PGE₂, thromboxane B₂, leukotriene C₄, and E₄, and thyroid hormones T4 and T3 ^{1,2}. OATPC has also been shown to be involved in the transport of xenobiotics , and drugs involved in lipid lowering *e.g.* statins¹. Statins have been refered to as a first-line therapy for patients with atherosclerotic vascular diseases. The OATPC gene and its product is also thought to be of importance in other diseases due to its transport of DHEAS an adrenal steroid which has been suggested to have positive neuropsychiatric, immune, and metabolic effects³. Due to the substrate specificity, location in the liver, and being exclusively expressed in the liver, Abe *et al* suggested that OATPC could be the predominant clearance mechanism of several endogenous and exogenous substrates in the liver. OATPC is the first human molecule reported to transport thyroid hormones².
¹ A Novel Human Hepatic Organic Anion Transporting Polypeptide (OATP2), Hsiang *et al* J Biol Chem **274**, 37161-37168 (1999)
² identification of a Novel Gene Family Encoding Human Liver-specifc Organic Anion Transporter LST-1, Takaaki Abe *et al* J Biol Chem **274,** 17159-17163 (1999)
³ Bates *et al* (1998) Curr. Opin. Endocrinol. Diab. **5**, 357-366

This liver specific transporter may be useful in liver-specific drug delivery systems and liver-specific chemotherapy, bile acid formation and the pathogenesis of diseases such as cholestasis, hyperbilirubinemia and thyroid hormone resistance.

The OATPC gene (sometimes called OAPT2 in the literature) has been cloned by four different groups, annotated and published as EMBL accession numbers AB026257 (OATPC, 2452bp), AF205071(OATP2, 2830, ref 1), AJ132573(OATP2, 2778)⁴, and AF060500 (LST-1)². Polymorphism has been reported by Tamai⁵ which is Asn130Asp and Val174Ala although any functional effect was stated therein to be not clear. Konig (2000) J Biol Chem 275, 23161-23168 describes the genomic organisation of OATP 1, 2 and 8. International patent application WO 00/08157 describes human anion transporter genes and some polymorphisms.
⁴ A novel human organic anion transporting polypeptide localised to the basolateral hepatocyte membrane, Konig Jorg *et al* (2000) Am J Physiol. Gastrointest. Liver Physiol. **278**: G156-G164
⁵Tamai *et al* (2000), BBRC, **273**, 251-60

All positions herein of polymorphisms in the OATPC polynucleotide relate to the position in one of SEQ ID NO 1 or 3-12 unless stated otherwise or apparent from the context.

All positions herein of polymorphisms in the OATPC polypeptide relate to the position in SEQ ID NO 2 unless stated otherwise or apparent from the context.

One approach is to use knowledge of polymorphisms to help identify patients most suited to therapy with particular pharmaceutical agents (this is often termed "pharmacogenetics") . Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder *et al.* (1997), Clinical Chemistry, **43**, 254; Marshall (1997), Nature Biotechnology, **15**, 1249; International Patent Application WO 97/40462, Spectra Biomedical; and Schafer *et al.* (1998), Nature Biotechnology, **16**, 33.

Clinical trials have shown that patient response to treatment with pharmaceuticals is often heterogeneous. Thus there is a need for improved approaches to pharmaceutical agent design and therapy.

Point mutations in polypeptides will be referred to as follows: natural amino acid (using 1 or 3 letter nomenclature) , position, new amino acid. For (a hypothetical) example "D25K" or "Asp25Lys" means that at position 25 an aspartic acid (D) has been changed to lysine (K). Multiple mutations in one polypeptide will be shown between square brackets with individual mutations separated by commas.

The present invention is based on the discovery of polymorphisms in OATPC.

According to one aspect of the present invention there is provided a method for the detection of a polymorphism in OATPC in a human, which method comprises determining the sequence of the human at at least one polymorphic position and determining the status of the human by reference to polymorphism in the OATPC gene. Preferred polymorphic positions are one or more of the following positions:
A method for the detection of a polymorphism in OATPC in a human, which method comprises determining the sequence of the human at at least one of the following polymorphic positions:
positions 510, 696, 1299, 1312, 1347, 1561, 2028, 2327 and 2342 in sequence of the OATPC gene as defined by the position in SEQ ID NO: 1;
positions 400, 405, 488 and 643 in OATPC polypeptide defined by position in SEQ ID NO: 2;
positions 321 and 1332 defined by position in SEQ ID NO 3;
position 41 defined by position in SEQ ID NO 4;
positions 109 and 244 defined by position in SEQ ID NO 5;
positions 117 and 283 defined by position in SEQ ID NO 6;
positions 209 and 211 defined by position in SEQ ID NO 7;
positions 63 to 68 defined by position in SEQ ID NO 8;
position 53 defined by position in SEQ ID NO 9;
position 75 defined by position in SEQ ID NO 10;
position 162 defined by position in SEQ ID NO 11; and
positions 84 defined by position in SEQ ID NO 12.

The term human includes both a human having or suspected of having a OATPC mediated disease and an asymptomatic human who may be tested for predisposition or susceptibility to such disease. At each position the human may be homozygous for an allele or the human may be a heterozygote.

The term polymorphism includes single nucleotide substitution, nucleotide insertion and nucleotide deletion which in the case of insertion and deletion includes insertion or deletion of one or more nucleotides at a position of a gene.

The method for diagnosis is preferably one in which the sequence is determined by a method selected from amplification refractory mutation system and restriction fragment length polymorphism.

The status of the individual may be determined by reference to allelic variation at any one, two, three, four, five, six, seven, eight, nine or more positions.

The test sample of nucleic acid is conveniently a sample of blood, bronchoalveolar lavage fluid, sputum, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on the PCR. These may be used in combination with a number of signal generation systems, a selection of which is listed in Table 2. Further amplification techniques are listed in Table 3. Many current methods for the detection of allelic variation are reviewed by Nollau *et al.,* Clin. Chem. **43**, 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

| **Abbreviations:** | |
|---|---|
| ALEX™ | Amplification refractory mutation system linear extension |
| APEX | Arrayed primer extension |
| ARMS™ | Amplification refractory mutation system |
| b-DNA | Branched DNA |
| bp | base pair |
| CMC | Chemical mismatch cleavage |
| COPS | Competitive oligonucleotide priming system |
| DGGE | Denaturing gradient gel electrophoresis |
| FRET | Fluorescence resonance energy transfer |
| HMG-CoA | 3-hydroxy-3-methylglutaryl-coenzyme A |
| LCR | Ligase chain reaction |
| MASDA | Multiple allele specific diagnostic assay |
| NASBA | Nucleic acid sequence based amplification |
| OATP | Na+-independent organic anion transporting polypeptide |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |

### Table 1 - Mutation Detection Techniques

**General:** DNA sequencing, Sequencing by hybridisation
**Scanning:** PTT*, SSCP, DGGE, TGGE, Cleavase, Heteroduplex analysis, CMC, Enzymatic mismatch cleavage

* Note: not useful for detection of promoter polymorphisms.

### Hybridisation Based

Solid phase hybridisation: Dot blots, MASDA, Reverse dot blots, Oligonucleotide arrays (DNA Chips)
Solution phase hybridisation: Taqman™ - US-5210015 & US-5487972 (Hoffinann-La Roche), Molecular Beacons - Tyagi *et al* (1996), Nature Biotechnology, **14**, 303; WO 95/13399 (Public Health Inst., New York)
**Extension Based:** ARMS™, ALEX™ - European Patent No. EP 332435 B1 (Zeneca Limited), COPS - Gibbs *et al* (1989), Nucleic Acids Research, **17**, 2347.
**Incorporation Based:** Mini-sequencing, APEX
**Restriction Enzyme Based**: RFLP, Restriction site generating PCR
**Ligation Based**: OLA
**Other:** Invader assay

### Table 2 - Signal Generation or Detection Systems

**Fluorescence:** FRET, Fluorescence quenching, Fluorescence polarisation - United Kingdom Patent No. 2228998 (Zeneca Limited)
**Other**: Chemiluminescence, Electrochemiluminescence, Raman, Radioactivity, Colorimetric, Hybridisation protection assay, Mass spectrometry

### Table 3 - Further Amplification Methods

### SSR, NASBA, LCR, SDA, b-DNA

Preferred mutation detection techniques include ARMS™, ALEX™, COPS, Taqman, Molecular Beacons, RFLP, and restriction site based PCR and FRET techniques.

Particularly preferred methods include ARMS™ and RFLP based methods. ARMS™ is an especially preferred method.

In a further aspect, the diagnostic methods of the invention are used to assess the pharmacogenetics of a drug transportable by OATPC.

Assays, for example reporter-based assays, may be devised to detect whether one or more of the above polymorphisms affect transcription levels and/or message stability.

Individuals who carry particular allelic variants of the OATPC gene may therefore exhibit differences in their ability to regulate protein biosynthesis under different physiological conditions and will display altered abilities to react to different diseases. In addition, differences arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition and/or susceptibility of an individual to diseases mediated by OATPC. This may be particularly relevant in the development of hyperlipoproteinemia and cardiovascular disease and the present invention may be used to recognise individuals who are particularly at risk from developing these conditions.

In a further aspect, the diagnostic methods of the invention are used in the development of new drug therapies which selectively target one or more allelic variants of the OATPC gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes.

According to another aspect of the present invention there is provided a human OATPC gene or its complementary strand comprising a variant allelic polymorphism at one or more of positions defined herein or a fragment thereof of at least 20 bases comprising at least one novel polymorphism.

Fragments are at least 17 bases, more preferably at least 20 bases, more preferably at least 30 bases.

According to another aspect of the present invention there is provided a polynucleotide comprising at least 20 bases of the human OATPC gene and comprising an allelic variant selected from any one of the following:

| Region | variant | Position in SEQ ID NO | SEQ ID NO |
|---|---|---|---|
| Exon 4 | A | 510 | 1 |
| Exon 5 | T | 670 | 1 |
| Exon 5 | T | 696 | 1 |
| Exon 9 | G | 1299 | 1 |
| Exon 9 | A | 1312 | 1 |
| Exon 9 | A | 1347 | 1 |
| Exon 10 | C | 1561 | 1 |
| Exon 14 | C | 2028 | 1 |
| 3'UTR | Insert T | 2327 | 1 |
| 3'UTR | C | 2342 | 1 |
| Promoter | G | 321 | 3 |
| Promoter | C | 1332 | 3 |
| Intron 1 | A | 41 | 4 |
| Intron 2 | G | 109 | 5 |
| Intron 2 | G | 244 | 5 |
| Intron 3 | A | 117 | 6 |
| Intron 3 | A | 283 | 6 |
| Intron 4 | A | 209 | 7 |
| Intron 4 | A | 211 | 7 |
| Intron 4 | Deletion CTTGTA | 63 | 8 |
| Intron 6 | T | 53 | 9 |
| Intron 9 | Insert TTC | 75 | 10 |
| Intron 11 | Insert T | 162 | 11 |
| Intron 12 | C | 84 | 12 |

According to another aspect of the present invention there is provided a human OATPC gene or its complementary strand comprising a polymorphism, preferably corresponding with one or more the positions defined herein or a fragment thereof of at least 20 bases comprising at least one polymorphism.

Fragments are at least 17 bases, more preferably at least 20 bases, more preferably at least 30 bases.

The invention further provides a nucleotide primer which can detect a polymorphism of the invention.

According to another aspect of the present invention there is provided an allele specific primer capable of detecting a OATPC gene polymorphism, preferably at one or more of the positions as defined herein.

An allele specific primer is used, generally together with a constant primer, in an amplification reaction such as a PCR reaction, which provides the discrimination between alleles through selective amplification of one allele at a particular sequence position e.g. as used for ARMS™ assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a OATPC gene polymorphism, preferably at one or more of the positions defined herein.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

According to another aspect of the present invention there is provided an allele specific primer or an allele specific oligonucleotide probe capable of detecting a OATPC gene polymorphism at one of the positions defined herein.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s) and polymerase(s) such as thermostable polymerases, for example taq polymerase.

In another aspect of the invention, the single nucleotide polymorphisms of this invention may be used as genetic markers in linkage studies. This particularly applies to the polymorphisms of relatively high frequency. The OATPC gene is on chromosome 12p (as shown from a database search with the cDNA as a query sequence). Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: Linkage disequilibrium at the cystathionine beta synthase (CBS) locus and the association between genetic variation at the CBS locus and plasma levels of homocysteine. *Ann Hum Genet* (1998) **62**:481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels: identification of three novel single nucleotide polymorphisms in the vWF gene promoter. *Blood* (1999) **93**:4277-83, Keightley AM, Lam YM, Brady JN, Cameron CL, Lillicrap D).

According to another aspect of the present invention there is provided a computer readable medium comprising at least one novel sequence of the invention stored on the medium. The computer readable medium may be used, for example, in homology searching, mapping, haplotyping, genotyping or pharmacogenetic analysis.

According to another aspect of the present invention there is provided a method of treating a human in need of treatment with a drug transportable by OATPC in which the method comprises:
i) detection of a polymorphism in OATPC in the human, which detection comprises determining the sequence of the human at one or more of the following positions:
   positions 487, 510,, 554, 670, 696, 819, 820, 1299, 1312, 1347, 1561, 2028, 2327 and 2342 in sequence of the OATPC gene as defined by the position in SEQ ID NO: 1;
   positions 130, 152, 174, 241, 400, 405, 488 and 643 in OATPC polypeptide defined by position in SEQ ID NO: 2;
   positions 321 and 1332 defined by position in SEQ ID NO 3;
   position 41 defined by position in SEQ ID NO 4;
   positions 109 and 244 defined by position in SEQ ID NO 5;
   positions 117 and 283 defined by position in SEQ ID NO 6;
   positions 209 and 211 defined by position in SEQ ID NO 7;
   positions 63 to 68 defined by position in SEQ ID NO 8;
   position 53 defined by position in SEQ ID NO 9;
   position 75 defined by position in SEQ ID NO 10;
   position 162 defined by position in SEQ ID NO 11; and
   positions 84 defined by position in SEQ ID NO 12.
   and determining the status of the human by reference to polymorphism in the OATPC gene; and
ii) administering an effective amount of the drug.

Preferably determination of the status of the human is clinically useful. Examples of clinical usefulness include deciding which statin drug or drugs to administer and/or in deciding on the effective amount of the statin drug or drugs. Statins already approved for use in humans include atorvastatin, cerivastatin, fluvastatin, pravastatin and simvastatin. The reader is referred to the following references for further information: Drugs and Therapy Perspectives (12^{th} May 1997), **9**: 1-6; Chong (1997) Pharmacotherapy **17**: 1157-1177; Kellick (1997) Formulary **32**: 352; Kathawala (1991) Medicinal Research Reviews, **11**: 121-146; Jahng (1995) Drugs of the Future **20**: 387-404, and Current Opinion in Lipidology, (1997), 8, 362 - 368. A preferred statin drug is compound 3a (S-4522) in Watanabe (1997) Bioorganic and Medicinal Chemistry **5:** 437-444; now called rosuvastatin, see Olsson (2001) American Journal of Cardiology, 87, supplement 1, 33-36. The term "drug transportable by OATPC" means that transport by OATPC in humans is an important part of a drug exerting its pharmceutical effect in man. For example, some statins have to be transported to the liver by OATPC to exert their lipid lowering effects.

According to another aspect of the present invention there is provided a method of treating a human in need of treatment with a drug transportable by OATPC in which the method comprises:
i) diagnosis of a single nucleotide polymorphism in OATPC gene in the human, which diagnosis preferably comprises determining the sequence of the nucleic acid at position 487 in the coding sequence of the OATPC gene as defined by the position in SEQ ID NO: 1. and determining the status of the human by reference to polymorphism in the OATPC gene; and
ii) administering an effective amount of the drug.

Although the polymorphism at position 487 (A→G; Asn130Asp) was reported by Tamai, no functional effect was attributed. Indeed the following polymorphisms are known but have not previously been attributed a role in statin pharmacogenetics as disclosed herein.

| **OATPC polymorphisms** | | | | | |
|---|---|---|---|---|---|
| Position of nucleotide as in sequence 1 of OATPC patent | | | | | |
| Position of amino acid as in sequence 2 of OATPC patent | | | | | |

| **Exon** | **nucleotide** | **SNP** | **Aminoacid** | **Change** | **Change** |
|---|---|---|---|---|---|
| 4 | 487 | A>G | 130 | Asn>Asp | N>D |
| 4 | 554 | G>A | 152 | Arg>Lys | R>K |
| 5 | 620 | T>C | 174 | Val>Ala | V>A |
| 5 | 670 | C>T | 191 | Leu | L |
| 6 | 819 | A>T | 240 | Val | V |
| 6 | 820 | G>A | 241 | Asp>Asn | D>N |

According to another aspect of the present invention there is provided use of a drug transportable by OATPC in preparation of a medicament for treating a disease in a human diagnosed as having a polymorphism therein, preferably at one or more of the positions defined herein. Preferably the disease is cardiovascular.

According to another aspect of the present invention there is provided a pharmaceutical pack comprising OATPC transportable drug and instructions for administration of the drug to humans diagnostically tested for a single nucleotide polymorphism therein, preferably at one or more of the positions defined herein.

According to another aspect of the present invention there is provided an allelic variant of human OATPC polypeptide comprising at least one of the following:
a leucine at position 400 of SEQ ID NO 2;
an isoleucine at position 405 of SEQ ID NO 2;
an arginine at position 488 of SEQ ID NO 2;
a phenylalanine at position 643 of SEQ ID NO 2;
or a fragment thereof comprising at least 10 amino acids provided that the fragment comprises at least one allelic variant.

Fragments of polypeptide are at least 10 amino acids, more preferably at least 15 amino acids, more preferably at least 20 amino acids.

According to another aspect of the present invention there is provided an antibody specific for an allelic variant of human OATPC polypeptide as described herein.

Antibodies can be prepared using any suitable method. For example, purified polypeptide may be utilized to prepare specific antibodies. The term "antibodies" is meant to include polycional antibodies, monoclonal antibodies, and the various types of antibody constructs such as for example F(ab')₂, Fab and single chain Fv. Antibodies are defined to be specifically binding if they bind the allelic variant of OATPC with a Kₐ of greater than or equal to about 10⁷ M⁻¹. Affinity of binding can be determined using conventional techniques, for example those described by Scatchard et al., *Ann. N.Y. Acad. Sci.,* 51:660 (1949).

Polyclonal antibodies can be readily generated from a variety of sources, for example, horses, cows, goats, sheep, dogs, chickens, rabbits, mice or rats, using procedures that are well-known in the art. In general, antigen is administered to the host animal typically through parenteral injection. The immunogenicity of antigen may be enhanced through the use of an adjuvant, for example, Freund's complete or incomplete adjuvant. Following booster immunizations, small samples of serum are collected and tested for reactivity to antigen. Examples of various assays useful for such determination include those described in: *Antibodies: A Laboratory Manual,* Harlow and Lane (eds.), Cold Spring Harbor Laboratory Press, 1988; as well as procedures such as countercurrent immuno-electrophoresis (CIEP), radioimmunoassay, radioimmunoprecipitation, enzyme-linked immuno-sorbent assays (ELISA), dot blot assays, and sandwich assays, see U.S. Patent Nos. 4,376,110 and 4,486,530.

Monoclonal antibodies may be readily prepared using well-known procedures, see for example, the procedures described in U.S. Patent Nos. RE 32,011, 4,902,614, 4,543,439 and 4,411,993; Monoclonal Antibodies, Hybridomas: *A New Dimension in Biological Analyses,* Plenum Press, Kennett, McKearn, and Bechtol (eds.), (1980).

The monoclonal antibodies of the invention can be produced using alternative techniques, such as those described by Alting-Mees et al., "Monoclonal Antibody Expression Libraries: A Rapid Alternative to Hybridomas", *Strategies in Molecular Biology* 3*:* 1-9 (1990) which is incorporated herein by reference. Similarly, binding partners can be constructed using recombinant DNA techniques to incorporate the variable regions of a gene that encodes a specific binding antibody. Such a technique is described in Larrick et al., *Biotechnology,* 7: 394 (1989).

Once isolated and purified, the antibodies may be used to detect the presence of antigen in a sample using established assay protocols, see for example "A Practical Guide to ELISA" by D. M. Kemeny, Pergamon Press, Oxford, England.

According to another aspect of the invention there is provided a diagnostic kit comprising an antibody of the invention.

The invention will now be illustrated but not limited by reference to the following Examples. All temperatures are in degrees Celsius.

In the Examples below, unless otherwise stated, the following methodology and materials have been applied.

AMPLITAQ™ ,available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989).

Electropherograms were obtained in a standard manner: data was collected by ABI377 data collection software and the wave form generated by ABI Prism sequencing analysis (2.1.2).

### Example 1

### Identification of Polymorphisms

### 1. Methods

### DNA Preparation

DNA was prepared from frozen blood samples collected in EDTA following protocol I (Molecular Cloning: A Laboratory Manual, p392, Sambrook, Fritsch and Maniatis, 2^{nd} Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water.

### Template Preparation

Templates were prepared by PCR using the oligonucleotide primers and annealing temperatures set out below. The extension temperature was 72° and denaturation temperature 94°. Generally 50 ng of genomic DNA was used in each reaction and subjected to 35 cycles of PCR. Where described below, the primary fragment was diluted 1/100 and two microlitres were used as template for amplification of secondary fragments. PCR was performed in two stages (primary fragment then secondary fragment) to ensure specific amplification of the desired target sequence.

| **Polymorphisms in OATPC: cDNA screening of 15 Liver samples** | | | | |
|---|---|---|---|---|
| **Region** | **SNP** | **Position** | **Amino Acid Change** | **Allele frequencies** |
| Exon 4 | G/A | 510 | None | G=96.7% |
| | | | | A=3.3% |
| Exon 5 | C/T | 670 | None | C=50% |
| | | | | T=50% |
| Exon 5 | C/T | 696 | None | C=60% |
| | | | | T=40% |
| Exon 9 | C/G | 1299 | Phe400Leu | C=96.7% |
| | | | | G=3.3% |
| Exon 9 | G/A | 1312 | Val405Ile | G=96.7% |
| | | | | A=3.3% |
| Exon 9 | G/A | 1347 | None | G=96.7% |
| | | | | A=3.3% |
| Exon 10 | G/C | 1561 | Gly488Arg | G=96.7% |
| | | | | C=3.3% |
| Exon 14 | A/C | 2028 | Leu643Phe | A=90% |
| | | | | C=10% |

OATP2 above refers to the clone sequenced by Hsiang et al (ref 1). Some comment on the numbering of exons in OATPC is required. This gene contains an exon (38 bp) upstream (5' UTR region) of the exon containing the ATG start site for translation. Therefore the exon numbering could vary depending whether this exon is counted as the first exon or not. In the literature, Konig (2000) JBC 275: 23161-68, have defined exon 1 as that containing the ATG start site and therefore we have adopted the same numbering in this application (but note that the priority document relating to the present application did vice versa; for example, exon 5 in this application is equivalent to exon 6 in the priority document).

| **PCR PRODUCTS** | | |
|---|---|---|
| **Fragment** | **Forward Oligo** | **Reverse Oligo** |
| 443-999 | 443-466 | 979-999 |
| 874-1360 | 874-896 | 1337-1360 |
| 1255-1684 | 1255-1278 | 1663-1684 |
| 1559-2095 | 1559-1581 | 2073-2095 |

| **RFLP analysis** | | | |
|---|---|---|---|
| **Polymorphism** | **Position** | **RFLP** **Enzyme/ PCR size** | **RFLP fragment size** |
| G/A | 510 | | |
| C/T | 670 | BmR I/ 595bp | C=349bp, 246bp |
| | | | T=595bp |
| C/T | 696 | | |
| C/G | 1299 | Apo I/ 595bp | C=30bp, 60bp, 380bp |
| | | | G=90bp, 380bp |
| G/A | 1312 | Bst 4CI/ 595bp | G=77bp, 393bp |
| | | | A=470bp |
| G/A | 1347 | | |
| G/C | 1561 | HpyCH4IV/595bp | G=470bp |
| | | | C=144bp, 326bp |
| A/C | 2028 | Ase I/577bp | A=89bp,488bp |
| | | | C=577bp |

### Example 2

| **Further OATPC Polymorphisms** | | | | | | | |
|---|---|---|---|---|---|---|---|
| **SNPs in OATPC 3'UTR** (positions according to SEQ ID NO 1) | | | | | | | |
| **Exon** | **Nucleotide** | **SNP** | | | | **Frequency** | **Frequency** |
| | | | | | | **Caucasian** | **Japanese** |
| 3' UTR | 2327 | Ins T | | | | not screened | 0.1 |
| 3' UTR | 2342 | T>C | | | | not screened | 0.4 |
| | | | | | | | |
| | | | | | | | |

| **SNPs in OATPC promoter** (positions according to SEQ ID NO 3) | | | | | | | |
|---|---|---|---|---|---|---|---|
| | **Nucleotide** | **SNP** | | | | **I Frequency** | **Frequency** |
| | | | | | | Caucasian | Japanese |
| | 321 | T>G | | | | 0.03 | not screened |
| | 1332 | A>C | | | | 0.08 | not screened |
| | | | | | | | |
| | | | | | | | |

| **SNPs in OATPC introns** | | | | | | | |
|---|---|---|---|---|---|---|---|
| **Intron** | **Nucleotide** | **SNP** | **Nucleotide** | **Sequence** | | | |
| | **position in** | | **position** | **ID No** | | | |
| | **relation to exon** | | **in sequence** | | | | |
| | | | | | | | |
| 1 | IVS1+21 | T>A | 41 | 4 | | | |
| 2 | IVS2+89 | T>G | 109 | 5 | | | |
| 2 | IVS2+224 | A>G | 244 | 5 | | | |
| 3 | IVS3+97 | C>A | 117 | 6 | | | |
| 3 | IVS3+263 | G>A | 283 | 6 | | | |
| 4 | IVS4+189 | G>A | 209 | 7 | | | |
| 4 | IVS4+191 | G>A | 211 | 7 | | | |
| 4 | IVS5-118 | deICTTGTA | 63 | 8 | | | |
| 6 | IVS6+33 | C>T | 53 | 9 | | | |
| 9 | IVS10-107 | ins TTC | 75 | 10 | | | |
| 11 | IVS11+142 | Ins T | 162 | 11 | | | |
| 12 | IVS13-97 | G>C | 84 | 12 | | | |

### OATPC intronic SNPs

### Key

20bp of exon sequence shown in uppercase
Intron sequence in lowercase (200 to 300bp only)
SNP shown in uppercase (one allele only)

Sequence ID No 4
IVS1+21     T>A          SNP at position 41 in this sequence

GATACTGCAA TGGATTGAAG gtagaataag ttttatgttt Ttgagctaaa ataagtaaat  60
agggaacttt aatgtataga aaagcaagtt gttaaaaaga acattatgtt tcaaattata  120
attttcaatt gaagcatata ttgaaatatt aacataatga ttcatacctt gatttaaacc  180
agtcttttaa tctgattaag  200

Sequence ID No 5
IVS2+89     T>G          SNP at position 109 in this sequence
IVS2+224    A>G          SNP at position 244 in this sequence

TGACGGAAGC TTTGAAATTG gtaacattta ttttctattt taataaccaa acttgcaaag  60
ttaaaaaata tatatgcttt acaccactgg ttatcaactg gggtaaatTt atctctcaca  120
ggcaatttgg caataactaa aaacatttgt ggttgtcata actgcacagg ggttgggggc  180
aatggaagtg ctactggtat ctaaaggtag aggtcagggg tactgctaaa tattctataa  240
tgcAcaaaga atgatgtaac tgaaaatgtt gatagtgagg atgttcagaa accctgattc  300

Sequence ID No 6
IVS3+97     C>A          SNP at position 117 in this sequence
IVS3+263    G>A          SNP at position 283 in this sequence

CCACATTTCT TCATGGGATA gtaagtgtta aaaaaaaaaa aaacctctgt gccactatca 60
gtaccttgta aattaggagt agaattttat tattatccct ttaaataggc agttacCttt 120
tgagaagata cccactaagt gtgtacagaa atgaaatagt gtctatttgt ctacataatc 180
attttattta tcgtagcttt catatacttt gaaataacaa aaagactaaa ctgtagagtt 240
tcaaatgaaa taaataggct ttttatgaat ttttagtata acGtatatac tgtacgtctt 300

Sequence ID No 7
IVS4+189    G>A    SNP at position 209 in this sequence
IVS4+191    G>A    SNP at position 211 in this sequence

ACCTGAGATA GTGGGAAAAG gtaagaatta atattgacag taaaaagtct tctaaaatgt  60
atacatttaa ttacatctct aaaaattgtt gtgatattca ttagcaaaat ttaattaaga  120
atgaatagga aaaacatttg actcttacag acataattat agtgttaata tacacagttc  180
gcccattaac aacacaggtt taaactacGc Gttttcactt ctatgcaaat tttgtccatc  240
tgaactggat gataaacctg ccggtaagaa tatctgacat tttctatatt tggattgaac  300

Sequence ID No 8
IVS5-118    delCTTGTA   deletion of 6bp from position 63 to 68 incl.

tagcagcata agaatggact aatacaccat attgtcaaag tttgcaaagt gaatataaat  60
taCTTGTAct tgtaaattaa aaaaaaataa gtagaataat taagagttta caagtagtta  120
aatttgtaat agaaatgcta aaattaatgt ttaaaatgaa acactctctt atctacatag  180
GTTGTTTAAA GGAATCTGGG  200

Sequence ID No 9IVS6+33 C>T          SNP at position 53 in this sequence
TATTGGATAT GTAGATCTAA gtaagtacaa ccagaacaag gtaccatgat aaCgtctttc  60
taagcacaca tgcgaaaaac attttttcaa ataactgaat tcactctttc aatagtcctt  120
tgcttaatat aattagaaag ttacaagtag gaaataaatg tattactaat cagaataaat  180
ataaaatcca gctcctattt  200

Sequence ID No 10
IVS10-107   Ins TTC     SNP at position 75 in this sequence
ttaaaaaaaa ctttgccatt tcgtcatcat caaagcaaat ttcttcatat aaagaaaaat  60
tctttatcta cttt(TTC)ttttcc ctctttctct gctttcactt tacttcttcc ttctcctccc  120
cttctttgtc tttttcttct ctctctctct ttttgatata tgtctatcat atatttccag  180
AAATAATCCA GTGACATCTC  200

Sequence ID No 11
IVS11+142    InsT        InsT at position 162 in this sequence

CATGTCATGC TGATTGTTAA gtaagtatga cttttaaaaa cattttcata tgcatgagac  60
tataaacaca cctaatgata tgcatatttt tacataatat actgggaatt caaattcata  120
tttcatcaaa ttttaatttt ctgagaattc attttattaa aa(T)ttactatg aactctcaag  180
gctgtaatta ataattttgc  200

Sequence ID No 12
IVS13-97    G>C         SNP at position 84 in this sequence

tgatttgggt ctttgagatt tctaataatc tttattattg ggtagatgca gaacaaaata  60
ataaacgaat cctccaaatt tttGaacttt tatttaatca aaatatatca atgtggaata  120
tcatgcagtt acatttaaaa tatgttccct aaactgacat cttctcttct cctattacag  180
GAGGAATTCT AGCTCCAATA  200

### OATPC promoter region

Total length of the sequence = 1538bp
1500bp of OATPC sequence directly upstream from the cDNA sequence
Sequence in uppercase represents 38bp overlap with the cDNA sequence (SEQ ID NO 1) where this 38bp is 5'UTR sequence.

Nucleotide positions in the promoter have been determined where the -1 position is the base (lowercase) directly upstream of the end of the cDNA sequence.

## Claims

1. A method for the detection of a polymorphism in OATPC in a human, which method comprises determining the sequence of the human at at least one of the following polymorphic positions:
positions 510, 696, 1299, 1312, 1347, 1561, 2028, 2327 and 2342 in sequence of the OATPC gene as defined by the position in SEQ ID NO: 1;
positions 400, 405, 488 and 643 in OATPC polypeptide defined by position in SEQ ID NO: 2;
positions 321 and 1332 defined by position in SEQ ID NO 3;
position 41 defined by position in SEQ ID NO 4;
positions 109 and 244 defined by position in SEQ ID NO 5;
positions 117 and 283 defined by position in SEQ ID NO 6;
positions 209 and 211 defined by position in SEQ ID NO 7;
positions 63 to 68 defined by position in SEQ ID NO 8;
position 53 defined by position in SEQ ID NO 9;
position 75 defined by position in SEQ ID NO 10;
position 162 defined by position in SEQ ID NO 11; and
positions 84 defined by position in SEQ ID NO 12.

2. Use of a method as defined in claim 1 to assess the pharmacogenetics of a drug transportable by OATPC.

3. A polynucleotide comprising at least 20 bases of the human OATPC gene and comprising an allelic variant selected from any one of the following:
| Region | variant | Position in SEQ ID NO | SEQ ID NO |
|---|---|---|---|
| Exon 4 | A | 510 | 1 |
| Exon 5 | T | 670 | 1 |
| Exon 5 | T | 696 | 1 |
| Exon 9 | G | 1299 | 1 |
| Exon 9 | A | 1312 | 1 |
| Exon 9 | A | 1347 | 1 |
| Exon 10 | C | 1561 | 1 |
| Exon 14 | C | 2028 | 1 |
| 3'UTR | Insert T | 2327 | 1 |
| 3'UTR | C | 2342 | 1 |
| Promoter | G | 321 | 3 |
| Promoter | C | 1332 | 3 |
| Intron 1 | A | 41 | 4 |
| Intron 2 | G | 109 | 5 |
| Intron 2 | G | 244 | 5 |
| Intron 3 | A | 117 | 6 |
| Intron 3 | A | 283 | 6 |
| Intron 4 | A | 209 | 7 |
| Intron 4 | A | 211 | 7 |
| Intron 4 | Deletion CTTGTA | 63 | 8 |
| Intron 6 | T | 53 | 9 |
| Intron 9 | Insert TTC | 75 | 10 |
| Intron 11 | Insert T | 162 | 11 |
| Intron 12 | C | 84 | 12 |

4. A nucleotide primer which can detect a polymorphism as defined in claim 1.

5. An allele specific primer capable of detecting a OATPC gene polymorphism as defined in claim 1.

6. An allele-specific oligonucleotide probe capable of detecting a OATPC gene polymorphism as defined in claim 1.

7. Use of an OATPC polymorphism as defined in claim 1 as a genetic marker in a linkage study.

8. Use of a drug transportable by OATPC in preparation of a medicament for treating a disease in a human detected as having a OATPC polymorphism at one or more of the following positions:
positions 487, 510, 554, 670, 696, 819, 820, 1299, 1312, 1347, 1561, 2028, 2327 and 2342 in sequence of the OATPC gene as defined by the position in SEQ ID NO: 1;
positions 130, 152, 174, 241, 400, 405, 488 and 643 in OATPC polypeptide defined by position in SEQ ID NO: 2;
positions 321 and 1332 defined by position in SEQ ID NO 3;
position 41 defined by position in SEQ ID NO 4;
positions 109 and 244 defined by position in SEQ ID NO 5;
positions 117 and 283 defined by position in SEQ ID NO 6;
positions 209 and 211 defined by position in SEQ ID NO 7;
positions 63 to 68 defined by position in SEQ ID NO 8;
position 53 defined by position in SEQ ID NO 9;
position 75 defined by position in SEQ ID NO 10;
position 162 defined by position in SEQ ID NO 11; and
positions 84 defined by position in SEQ ID NO 12.

9. A use according to claim 10 in which the drug is a statin.

10. A use according to claim 10 in which the drug is rosuvastatin.

11. An allelic variant of human OATPC polypeptide comprising at least one of the following:
a leucine at position 400 of SEQ ID NO 2;
an isoleucine at position 405 of SEQ ID NO 2;
an arginine at position 488 of SEQ ID NO 2;
a phenylalanine at position 643 of SEQ ID NO 2;
or a fragment thereof comprising at least 10 amino acids provided that the fragment comprises at least one allelic variant.

12. An antibody specific for an allelic variant of human OATPC polypeptide as defined in claim 11.


SEQUENCE LISTING

<110> AstraZeneca AB

<120> Chemical Compounds

<130> adeokun
      ambrose
      cresswell
      dudley

<140>
<141>

<160> 12

<170> PatentIn Ver. 2.1

<210> 1
<211> 2452
<212> DNA
<213> Homo sapiens

<400> 1
gtggacttgt tgcagttgct gtaggattct aaatccaggt gattgtttca aactgagcat 60
caacaacaaa aacatttgta tgatatctat atttcaatca tggaccaaaa tcaacatttg 120
aataaaacag cagaggcaca accttcagag aataagaaaa caagatactg caatggattg 180
aagatgttct tggcagctct gtcactcagc tttattgcta agacactagg tgcaattatt 240
atgaaaagtt ccatcattca tatagaacgg agatttgaga tatcctcttc tcttgttggt 300
tttattgacg gaagctttga aattggaaat ttgcttgtga ttgtatttgt gagttacttt 360
ggatccaaac tacatagacc aaagttaatt ggaatcggtt gtttcattat gggaattgga 420
ggtgttttga ctgctttgcc acatttcttc atgggatatt acaggtattc taaagaaact 480
aatatcaatt catcagaaaa ttcaacatcg accttatcca cttgtttaat taatcaaatt 540
ttatcactca atagagcatc acctgagata gtgggaaaag gttgtttaaa ggaatctggg 600
tcatacatgt ggatatatgt gttcatgggt aatatgcttc gtggaatagg ggagactccc 660
atagtaccac tggggctttc ttacattgat gatttcgcta aagaaggaca ttcttctttg 720
tatttaggta tattgaatgc aatagcaatg attggtccaa tcattggctt taccctggga 780
tctctgtttt ctaaaatgta cgtggatatt ggatatgtag atctaagcac tatcaggata 840
actcctactg attctcgatg ggttggagct tggtggctta atttccttgt gtctggacta 900
ttctccatta tttcttccat accattcttt ttcttgcccc aaactccaaa taaaccacaa 960
aaagaaagaa aagcttcact gtctttgcat gtgctggaaa caaatgatga aaaggatcaa 1020
acagctaatt tgaccaatca aggaaaaaat attaccaaaa atgtgactgg tttttttcag 1080
tcttttaaaa gcatccttac taatcccctg tatgttatgt ttgtgctttt gacgttgtta 1140
caagtaagca gctatattgg tgcttttact tatgtcttca aatacgtaga gcaacagtat 1200
ggtcagcctt catctaaggc taacatctta ttgggagtca taaccatacc tatttttgca 1260
agtggaatgt ttttaggagg atatatcatt aaaaaattca aactgaacac cgttggaatt 1320
gccaaattct catgttttac tgctgtgatg tcattgtcct tttacctatt atattttttc 1380
atactctgtg aaaacaaatc agttgccgga ctaaccatga cctatgatgg aaataatcca 1440
gtgacatctc atagagatgt accactttct tattgcaact cagactgcaa ttgtgatgaa 1500
agtcaatggg aaccagtctg tggaaacaat ggaataactt acatctcacc ctgtctagca 1560
ggttgcaaat cttcaagtgg caataaaaag cctatagtgt tttacaactg cagttgtttg 1620
gaagtaactg gtctccagaa cagaaattac tcagcccatt tgggtgaatg cccaagagat 1680
gatgcttgta caaggaaatt ttactttttt gttgcaatac aagtcttgaa tttatttttc 1740
tctgcacttg gaggcacctc acatgtcatg ctgattgtta aaattgttca acctgaattg 1800
aaatcacttg cactgggttt ccactcaatg gttatacgag cactaggagg aattctagct 1860
ccaatatatt ttggggctct gattgataca acgtgtataa agtggtccac caacaactgt 1920
ggcacacgtg ggtcatgtag gacatataat tccacatcat tttcaagggt ctacttgggc 1980
ttgtcttcaa tgttaagagt ctcatcactt gttttatata ttatattaat ttatgccatg 2040
aagaaaaaat atcaagagaa agatatcaat gcatcagaaa atggaagtgt catggatgaa 2100
gcaaacttag aatccttaaa taaaaataaa cattttgtcc cttctgctgg ggcagatagt 2160
gaaacacatt gttaagggga gaaaaaaagc cacttctgct tctgtgtttc caaacagcat 2220
tgcattgatt cagtaagatg ttatttttga ggagttcctg gtcctttcac taagaatttc 2280
cacatctttt atggtggaag tataaataag cctatgaact tataataaaa caaactgtag 2340
gtagaaaaaa tgagagtact cattgtacat tatagctaca tatttgtggt taaggttaga 2400
ctatatgatc catacaaatt aaagtgagag acatggttac tgtgtaataa aa 2452

<210> 2
<211> 691
<212> PRT
<213> Homo sapiens

<400> 2
Met Asp Gln Asn Gln His Leu Asn Lys Thr Ala Glu Ala Gln Pro Ser
1               5                   10                  15

Glu Asn Lys Lys Thr Arg Tyr Cys Asn Gly Leu Lys Met Phe Leu Ala
            20                  25                  30

Ala Leu Ser Leu Ser Phe Ile Ala Lys Thr Leu Gly Ala Ile Ile Met
        35                  40                  45

Lys Ser Ser Ile Ile His Ile Glu Arg Arg Phe Glu Ile Ser Ser Ser
    50                  55                  60

Leu Val Gly Phe Ile Asp Gly Ser Phe Glu Ile Gly Asn Leu Leu Val
65                  70                  75                  80

Ile Val Phe Val Ser Tyr Phe Gly Ser Lys Leu His Arg Pro Lys Leu
                85                  90                  95

Ile Gly Ile Gly Cys Phe Ile Met Gly Ile Gly Gly Val Leu Thr Ala
            100                 105                 110

Leu Pro His Phe Phe Met Gly Tyr Tyr Arg Tyr Ser Lys Glu Thr Asn
        115                 120                 125

Ile Asn Ser Ser Glu Asn Ser Thr Ser Thr Leu Ser Thr Cys Leu Ile
    130                 135                 140

Asn Gln Ile Leu Ser Leu Asn Arg Ala Ser Pro Glu Ile Val Gly Lys
145                 150                 155                 160

Gly Cys Leu Lys Glu Ser Gly Ser Tyr Met Trp Ile Tyr Val Phe Met
                165                 170                 175

Gly Asn Met Leu Arg Gly Ile Gly Glu Thr Pro Ile Val Pro Leu Gly
            180                 185                 190

Leu Ser Tyr Ile Asp Asp Phe Ala Lys Glu Gly His Ser Ser Leu Tyr
        195                 200                 205

Leu Gly Ile Leu Asn Ala Ile Ala Met Ile Gly Pro Ile Ile Gly Phe
    210                 215                 220

Thr Leu Gly Ser Leu Phe Ser Lys Met Tyr Val Asp Ile Gly Tyr Val
225                 230                 235                 240

Asp Leu Ser Thr Ile Arg Ile Thr Pro Thr Asp Ser Arg Trp Val Gly
                245                 250                 255

Ala Trp Trp Leu Asn Phe Leu Val Ser Gly Leu Phe Ser Ile Ile Ser
            260                 265                 270

Ser Ile Pro Phe Phe Phe Leu Pro Gln Thr Pro Asn Lys Pro Gln Lys
        275                 280                 285

Glu Arg Lys Ala Ser Leu Ser Leu His Val Leu Glu Thr Asn Asp Glu
    290                 295                 300

Lys Asp Gln Thr Ala Asn Leu Thr Asn Gln Gly Lys Asn Ile Thr Lys
305                 310                 315                 320

Asn Val Thr Gly Phe Phe Gln Ser Phe Lys Ser Ile Leu Thr Asn Pro
                325                 330                 335

Leu Tyr Val Met Phe Val Leu Leu Thr Leu Leu Gln Val Ser Ser Tyr
            340                 345                 350

Ile Gly Ala Phe Thr Tyr Val Phe Lys Tyr Val Glu Gln Gln Tyr Gly
        355                 360                 365

Gln Pro Ser Ser Lys Ala Asn Ile Leu Leu Gly Val Ile Thr Ile Pro
    370                 375                 380

Ile Phe Ala Ser Gly Met Phe Leu Gly Gly Tyr Ile Ile Lys Lys Phe
385                 390                 395                 400

Lys Leu Asn Thr Val Gly Ile Ala Lys Phe Ser Cys Phe Thr Ala Val
                405                 410                 415

Met Ser Leu Ser Phe Tyr Leu Leu Tyr Phe Phe Ile Leu Cys Glu Asn
            420                 425                 430

Lys Ser Val Ala Gly Leu Thr Met Thr Tyr Asp Gly Asn Asn Pro Val
        435                 440                 445

Thr Ser His Arg Asp Val Pro Leu Ser Tyr Cys Asn Ser Asp Cys Asn
    450                 455                 460

Cys Asp Glu Ser Gln Trp Glu Pro Val Cys Gly Asn Asn Gly Ile Thr
465                 470                 475                 480

Tyr Ile Ser Pro Cys Leu Ala Gly Cys Lys Ser Ser Ser Gly Asn Lys
                485                 490                 495

Lys Pro Ile Val Phe Tyr Asn Cys Ser Cys Leu Glu Val Thr Gly Leu
            500                 505                 510

Gln Asn Arg Asn Tyr Ser Ala His Leu Gly Glu Cys Pro Arg Asp Asp
        515                 520                 525

Ala Cys Thr Arg Lys Phe Tyr Phe Phe Val Ala Ile Gln Val Leu Asn
    530                 535                 540

Leu Phe Phe Ser Ala Leu Gly Gly Thr Ser His Val Met Leu Ile Val
545                 550                 555                 560

Lys Ile Val Gln Pro Glu Leu Lys Ser Leu Ala Leu Gly Phe His Ser
                565                 570                 575

Met Val Ile Arg Ala Leu Gly Gly Ile Leu Ala Pro Ile Tyr Phe Gly
            580                 585                 590

Ala Leu Ile Asp Thr Thr Cys Ile Lys Trp Ser Thr Asn Asn Cys Gly
        595                 600                 605

Thr Arg Gly Ser Cys Arg Thr Tyr Asn Ser Thr Ser Phe Ser Arg Val
    610                 615                 620

Tyr Leu Gly Leu Ser Ser Met Leu Arg Val Ser Ser Leu Val Leu Tyr
625                 630                 635                 640

Ile Ile Leu Ile Tyr Ala Met Lys Lys Lys Tyr Gln Glu Lys Asp Ile
                645                 650                 655

Asn Ala Ser Glu Asn Gly Ser Val Met Asp Glu Ala Asn Leu Glu Ser
            660                 665                 670

Leu Asn Lys Asn Lys His Phe Val Pro Ser Ala Gly Ala Asp Ser Glu
        675                 680                 685

Thr His Cys

    690

<210> 3
<211> 1538
<212> DNA
<213> Homo sapiens

<400> 3
atgctctttg acctctgaaa atattggaga attttacaac tggcaccttt agctcaggat 60
tataaaggtt gttagttagt ttgtactgtt ttatcttcat tgtatataat atatatatta 120
gtctccaaac atgttgatgt gttttcaatg aaatggatgt ctgaggagaa aaccattagc 180
ctgagaaaac ccaaactgta ttcccattgt gaataaaagg aagtccataa aaatgatgga 240
aaatgttctg cattcctgtt atgatatcaa aatctggcag tacatgaaaa tttttcaaag 300
tgcttattta acaggcataa tctttggtct cctgagccag aatctgctgg gtatgggact 360
ggattgctat tttgacaact cgccagtaga ttcttactca gcagagtatt tggaagcctt 420
actctaatat tttggccttg ggtctacatt tctcagttct gcacagtcat tcttcccctc 480
tacactactc tttagtttgt ctcatgattc caatactctc aataattaac caagaataga 540
actaatcaat cagataactg tggcacagac atcaaataca ttttgctgca accatatcaa 600
caaatgtccc atgaatgata aggggtaacc atattctcat atatgcatcc tcacattacc 660
acatatatat atgtgcatat gtgtatacag gtaaaagtgt gtatatatgt atacatgtat 720
gtttgtgtgt atatacatac atatatcttc acacttttct gaaatatata tatttatgtg 780
agagaagggt ctgtacttta tttcagaaga gagcttaatg tccaaggtat aattgagagt 840
ctaaaatgtt tgagttattg aattaattaa acttcatctc tactcaagaa aacttttaac 900
tgagttaagc tcttcctttc tccacaagtc aagtcaataa aaggaaactg tgatattaat 960
aattctttcc tgttttgatg taaagaatct atcgcataaa gcagtcttaa ttttcatcat 1020
tcagaaaaat ggtcttgcag ttaattggga ctctcttatt ccaggtggta tctccagtct 1080
ccatacatac cacgttagaa ccatacttat gtaccaagca aagagggtat attttaattt 1140
ttaaatgcca atgtaacctg taggcatatt ttttatttgt cttaaattat ttcctatttg 1200
gaagttttaa atacctggaa taatttattg tactcatatt tttaaagaaa aaaatcttat 1260
gccaccaact taattgaata aacaagtaaa agccattccc aaaagtaagg tttacttgtt 1320
aagattaaca aaaaataatg tgagaattct gagaaatata atctttaaat attggcaact 1380
ggagtgaact cttaaaacta actaggtttt atatgtttga ctagagcaat gacataataa 1440
ggtggttaat catcactgga cttgttttca aaaagccaac tactttaaga ggaataaagg 1500
gtggacttgt tgcagttgct gtaggattct aaatccag 1538

<210> 4
<211> 200
<212> DNA
<213> Homo sapiens

<400> 4
gatactgcaa tggattgaag gtagaataag ttttatgttt ttgagctaaa ataagtaaat 60
agggaacttt aatgtataga aaagcaagtt gttaaaaaga acattatgtt tcaaattata 120
attttcaatt gaagcatata ttgaaatatt aacataatga ttcatacctt gatttaaacc 180
agtcttttaa tctgattaag 200

<210> 5
<211> 300
<212> DNA
<213> Homo sapiens

<400> 5
tgacggaagc tttgaaattg gtaacattta ttttctattt taataaccaa acttgcaaag 60
ttaaaaaata tatatgcttt acaccactgg ttatcaactg gggtaaattt atctctcaca 120
ggcaatttgg caataactaa aaacatttgt ggttgtcata actgcacagg ggttgggggc 180
aatggaagtg ctactggtat ctaaaggtag aggtcagggg tactgctaaa tattctataa 240
tgcacaaaga atgatgtaac tgaaaatgtt gatagtgagg atgttcagaa accctgattc 300

<210> 6
<211> 300
<212> DNA
<213> Homo sapiens

<400> 6
   ccacatttct tcatgggata gtaagtgtta aaaaaaaaaa aaacctctgt gccactatca 60
   gtaccttgta aattaggagt agaattttat tattatccct ttaaataggc agttaccttt 120
   tgagaagata cccactaagt gtgtacagaa atgaaatagt gtctatttgt ctacataatc 180
   attttattta tcgtagcttt catatacttt gaaataacaa aaagactaaa ctgtagagtt 240
   tcaaatgaaa taaataggct ttttatgaat ttttagtata acgtatatac tgtacgtctt 300

<210> 7
<211> 300
<212> DNA
<213> Homo sapiens

<400> 7
acctgagata gtgggaaaag gtaagaatta atattgacag taaaaagtct tctaaaatgt 60
atacatttaa ttacatctct aaaaattgtt gtgatattca ttagcaaaat ttaattaaga 120
atgaatagga aaaacatttg actcttacag acataattat agtgttaata tacacagttc 180
gcccattaac aacacaggtt taaactacgc gttttcactt ctatgcaaat tttgtccatc 240
tgaactggat gataaacctg ccggtaagaa tatctgacat tttctatatt tggattgaac 300

<210> 8
<211> 200
<212> DNA
<213> Homo sapiens

<400> 8
tagcagcata agaatggact aatacaccat attgtcaaag tttgcaaagt gaatataaat 60
tacttgtact tgtaaattaa aaaaaaataa gtagaataat taagagttta caagtagtta 120
aatttgtaat agaaatgcta aaattaatgt ttaaaatgaa acactctctt atctacatag 180
gttgtttaaa ggaatctggg 200

<210> 9
<211> 200
<212> DNA
<213> Homo sapiens

<400> 9
tattggatat gtagatctaa gtaagtacaa ccagaacaag gtaccatgat aacgtctttc 60
taagcacaca tgcgaaaaac attttttcaa ataactgaat tcactctttc aatagtcctt 120
tgcttaatat aattagaaag ttacaagtag gaaataaatg tattactaat cagaataaat 180
ataaaatcca gctcctattt 200

<210> 10
<211> 203
<212> DNA
<213> Homo sapiens

<400> 10
ttaaaaaaaa ctttgccatt tcgtcatcat caaagcaaat ttcttcatat aaagaaaaat 60
tctttatcta ctttttcttt tccctctttc tctgctttca ctttacttct tccttctcct 120
cccttctttt gtctttttct tctctctctc tctttttgat atatgtctat catatatttc 180
cagaaataat ccagtgacat ctc 203

<210> 11
<211> 201
<212> DNA
<213> Homo sapiens

<400> 11
   catgtcatgc tgattgttaa gtaagtatga cttttaaaaa cattttcata tgcatgagac 60
   tataaacaca cctaatgata tgcatatttt tacataatat actgggaatt caaattcata 120
   tttcatcaaa ttttaatttt ctgagaattc attttattaa aatttactat gaactctcaa 180
   ggctgtaatt aataattttg c 201

<210> 12
<211> 200
<212> DNA
<213> Homo sapiens

<400> 12
tgatttgggt ctttgagatt tctaataatc tttattattg ggtagatgca gaacaaaata 60
ataaacgaat cctccaaatt tttgaacttt tatttaatca aaatatatca atgtggaata 120
tcatgcagtt acatttaaaa tatgttccct aaactgacat cttctcttct cctattacag 180
gaggaattct agctccaata 200

atgctctttg acctctgaaa atattggaga attttacaac tggcaccttt agctcaggat 60
tataaaggtt gttagttagt ttgtactgtt ttatcttcat tgtatataat atatatatta 120
gtctccaaac atgttgatgt gttttcaatg aaatggatgt ctgaggagaa aaccattagc 180
ctgagaaaac ccaaactgta ttcccattgt gaataaaagg aagtccataa aaatgatgga 240
aaatgttctg cattcctgtt atgatatcaa aatctggcag tacatgaaaa tttttcaaag 300
tgcttattta acaggcataa tctttggtct cctgagccag aatctgctgg gtatgggact 360
ggattgctat tttgacaact cgccagtaga ttcttactca gcagagtatt tggaagcctt 420
actctaatat tttggccttg ggtctacatt tctcagttct gcacagtcat tcttcccctc 480
tacactactc tttagtttgt ctcatgattc caatactctc aataattaac caagaataga 540
actaatcaat cagataactg tggcacagac atcaaataca ttttgctgca accatatcaa 600
caaatgtccc atgaatgata aggggtaacc atattctcat atatgcatcc tcacattacc 660
acatatatat atgtgcatat gtgtatacag gtaaaagtgt gtatatatgt atacatgtat 720
gtttgtgtgt atatacatac atatatcttc acacttttct gaaatatata tatttatgtg 780
agagaagggt ctgtacttta tttcagaaga gagcttaatg tccaaggtat aattgagagt 840
ctaaaatgtt tgagttattg aattaattaa acttcatctc tactcaagaa aacttttaac 900
tgagttaagc tcttcctttc tccacaagtc aagtcaataa aaggaaactg tgatattaat 960
aattctttcc tgttttgatg taaagaatct atcgcataaa gcagtcttaa ttttcatcat 1020
tcagaaaaat ggtcttgcag ttaattggga ctctcttatt ccaggtggta tctccagtct 1080
ccatacatac cacgttagaa ccatacttat gtaccaagca aagagggtat attttaattt 1140
ttaaatgcca atgtaacctg taggcatatt ttttatttgt cttaaattat ttcctatttg 1200
gaagttttaa atacctggaa taatttattg tactcatatt tttaaagaaa aaaatcttat 1260
gccaccaact taattgaata aacaagtaaa agccattccc aaaagtaagg tttacttgtt 1320
aagattaaca aaaaataatg tgagaattct gagaaatata atctttaaat attggcaact 1380
ggagtgaact cttaaaacta actaggtttt atatgtttga ctagagcaat gacataataa 1440
ggtggttaat catcactgga cttgttttca aaaagccaac tactttaaga ggaataaagg 1500
GTGGACTTGT TGCAGTTGCT GTAGGATTCT AAATCCAG 1538